# EUROPEAN PATENT SPECIFICATION

(11) **EP 3 390 589 B1**
(45) Date of publication and mention of the grant of the patent: **23.02.2022**
(21) Application number: 16838041.8
(22) Date of filing: 19.12.2016
(51) Int. Cl.: C10L 1/185, B01J 4/00

(54) **GTBE COMPOSITIONS, METHODS AND INSTALLATIONS FOR ENHANCED OCTANE BOOSTING**
GTBE ZUSAMMENSETZUNGEN, HERSTELLUNGSMETHODEN UND EINRICHTUNGEN ZUR VERBESSERUNG DER OCTANZAHLERHÖHUNG
COMPOSITIONS DE GTBE ET DES MÉTHODES ET APPAREILS À PRODODUIRE GTBE POUR AUGMENTER L'INDICE D'OCTANE

(30) Priority: 18.12.2015 CA 2915852
(43) Date of publication of application: 24.10.2018
(73) Proprietor: Procede Holding B.V., 7544 GG Enschede (NL)
(72) Inventor: VERSTEEG, Geert Frederik, 7500 AH Enschede (NL); WERMINK, Wouter Nicolaas, 7500 AH Enschede (NL)
(74) Representative: V.O.
(86) International application number: PCT/NL2016/050889
(87) International publication number: WO 2017/105246

(56) References cited:
- DE-A1- 4 445 635
- DE-A1- 4 445 635
- DE-A1- 4 445 635
- US-A1- 2011 098 510
- US-A1- 2011 098 510
- US-A1- 2011 098 510
- Der ET AL: "Synthesis of Glycerol Based Fuel Additives to Reduce NOx Emissions from Diesel Engines Operated on Diesel and Biodiesel fuels by SNCR i s s e r t a t i o n", , 28 September 2010 (2010-09-28), XP055361600, Retrieved from the Internet: URL:http://rzbl04.biblio.etc.tu-bs.de:8080 /docportal/servlets/MCRFileNodeServlet/Doc Portal_derivate_00016392/Thesis.pdf [retrieved on 2017-04-04]
- MARTINO DI SERIO ET AL: "New Process for the Production of Glycerol tert -Butyl Ethers", ENERGY & FUELS., vol. 24, no. 9, 16 September 2010 (2010-09-16), pages 4668-4672, XP055676828, WASHINGTON, DC, US. ISSN: 0887-0624, DOI: 10.1021/ef901230r
- Shravan Kumar Tanugula et al: "Synthesis of Glycerol Based Fuel Additives to Reduce NOx Emissions from Diesel Engines Operated on Diesel and Biodiesel fuels by SNCR Di s s e r t a t i o n", , 28 September 2010 (2010-09-28), XP055361600, Retrieved from the Internet: URL:http://rzbl04.biblio.etc.tu-bs.de:8080 /docportal/servlets/MCRFileNodeServlet/Doc Portal_derivate_00016392/Thesis.pdf [retrieved on 2017-04-04]
- Der ET AL: "Synthesis of Glycerol Based Fuel Additives to Reduce NOx Emissions from Diesel Engines Operated on Diesel and Biodiesel fuels by SNCR i s s e r t a t i o n", , 28 September 2010 (2010-09-28), XP055361600, Retrieved from the Internet: URL:http://rzbl04.biblio.etc.tu-bs.de:8080 /docportal/servlets/MCRFileNodeServlet/Doc Portal_derivate_00016392/Thesis.pdf [retrieved on 2017-04-04]
- MARTINO DI SERIO ET AL: "New Process for the Production of Glycerol tert -Butyl Ethers", ENERGY & FUELS., vol. 24, no. 9, 16 September 2010 (2010-09-16), pages 4668-4672, XP055676828, WASHINGTON, DC, US. ISSN: 0887-0624, DOI: 10.1021/ef901230r

## Description

### TECHNICAL FIELD

The technical field generally relates to octane boosting for gasoline.

### BACKGROUND

There are various compounds that have been used for octane boosting. For example, blends of Glycerol Tert-Butyl Ethers (GTBE) have been used to boost octane for gasoline. These GTBE blends have consisted of mixtures of mono-, di- and tri-GTBE and have been able to achieve limited octane booster/enhancer ability.

### SUMMARY

Disclosed herein is a method of boosting octane of gasoline, comprising: contacting glycerol and isobutene in a reaction vessel under conditions to generate a blend of Glycerol Tert-Butyl Ethers (GTBE) comprising mono-GTBEs, di-GTBEs and tri-GTBE; regulating the conditions to favor production of mono-GTBEs according to equilibrium reactions, thereby producing a mono-shifted GTBE blend; and using the mono-shifted GTBE blend with gasoline as an octane booster.

The step of regulating may comprise controlling molar ratios of the glycerol and the isobutene. Controlling the molar ratio of isobutene/ glycerol may be done between 1/4 and 4/1. The molar ratio of isobutene/glycerol may be between 1/3 and 3/1. The molar ratio of isobutene/glycerol may be between 1/2.5 and 2.5/1. The molar ratio of isobutene/glycerol may be between 1/2 and 2/1. Controlling the molar ratio of isobutene/ glycerol may be done between 1/1 and 4/1. The molar ratio of isobutene/glycerol may be between 1.5/ and 2.5/1. The molar ratio of isobutene/glycerol may be between 1.75/ and 2.25/1. The molar ratio of isobutene/glycerol may be between 1.9/ and 2.1/1.

The mono-shifted GTBE blend can be optionally increased in mono-GTBE content by the use of a suitable separation step.

The molar ratio of isobutene/glycerol may be regulated to maximize production of mono-GTBEs relative to di-GTBEs and tri-GTBE.

The molar ratio of isobutene/glycerol, reaction temperature, and residence time may be provided to obtain a yield of the mono-GTBE of at least 10%. The molar ratio of isobutene/glycerol, reaction temperature, and residence time may be provided to obtain a yield of the mono-GTBE of at least 15%. The molar ratio of isobutene/glycerol, reaction temperature, and residence time may be provided to obtain a yield of the mono-GTBE of at least 20%. The molar ratio of isobutene/glycerol, reaction temperature, and residence time may be provided to obtain a yield of the mono-GTBE of at least 25%. The molar ratio of isobutene/glycerol, reaction temperature, and residence time may be provided to obtain a yield of the mono-GTBE of at least 30%. The molar ratio of isobutene/glycerol, reaction temperature, and residence time may be provided to obtain a yield of the mono-GTBE of at least 35%. The molar ratio of isobutene/glycerol, reaction temperature, and residence time may be provided to obtain a yield of the mono-GTBE of at least 40%. The molar ratio of isobutene/glycerol, reaction temperature, and residence time may be provided to obtain a yield of the mono-GTBE of at least 45%. The molar ratio of isobutene/glycerol, reaction temperature, and residence time may be provided to obtain a yield of the mono-GTBE between 20% and 50% or between 20% and 35%

The reaction temperature may be provided between 40°C and 100°C. The reaction temperature may be provided between 45°C and 95°C. The reaction temperature may be provided between 50°C and 90°C. The reaction temperature may be provided between 55°C and 85°C. The reaction temperature may be provided between 40°C and 85°C. The reaction temperature may be provided between 45°C and 80°C. The reaction temperature may be provided between 50°C and 75°C. The reaction temperature may be provided between 55°C and 70°C.

The mono-GTBE content of the GTBE exiting the reactor can be increased by the use of a suitable separation step, e.g. via extraction or (vacuum) distillation.

Depending on the regulation of the reactor settings for the production of the GTBEs, the concentration of the mono-GTBEs in the mono-GTBE, di-GTBE and tri-GTBE mixture may be increased to 15% by the use of a suitable separation step. Depending on the regulation of the reactor settings for the production of the GTBEs, the concentration of the mono-GTBEs in the mono-GTBE, di-GTBE and tri-GTBE mixture may be increased to 20% by the use of a suitable separation step. Depending on the regulation of the reactor settings for the production of the GTBEs, the concentration of the mono-GTBEs in the mono-GTBE, di-GTBE and tri-GTBE mixture may be increased to 40% by the use of a suitable separation step. Depending on the regulation of the reactor settings for the production of the GTBEs, the concentration of the mono-GTBEs in the mono-GTBE, di-GTBE and tri-GTBE mixture may be increased to 60% by the use of a suitable separation step. Depending on the regulation of the reactor settings for the production of the GTBEs, the concentration of the mono-GTBEs in the mono-GTBE, di-GTBE and tri-GTBE mixture may be increased to 80% by the use of a suitable separation step. Depending on the regulation of the reactor settings for the production of the GTBEs, the concentration of the mono-GTBEs in the mono-GTBE, di-GTBE and tri-GTBE mixture may be increased between 20% to 100% by the use of a suitable separation step.

Also disclosed herein is an octane boosting composition comprising Glycerol Tert-Butyl Ethers (GTBE) consisting of 3-tert-Butoxypropane-1,2-diol and 2-tert-Butoxypropane-1,3-diol.

The octane boosting composition may consist of mono-GTBEs, di-GTBEs and tri-GTBE, wherein the composition is formulated such that a concentration of the mono-GTBEs therein is sufficient to increase the octane number by at least 0.1 compared to a composition consisting of di-GTBEs and tri-GTBE.

The concentration of the mono-GTBEs may be sufficient to increase the octane number by at least 1.0 compared to the composition consisting of di-GTBEs and tri-GTBE. The concentration of the mono-GTBEs may be sufficient to increase the octane number by at least 2.0 compared to the composition consisting of di-GTBEs and tri-GTBE. The concentration of the mono-GTBEs may be sufficient to increase the octane number by at least 3.0 compared to the composition consisting of di-GTBEs and tri-GTBE. The concentration of the mono-GTBEs may be sufficient to increase the octane number by at least 4.0 compared to the composition consisting of di-GTBEs and tri-GTBE. The concentration of the mono-GTBEs may be sufficient to increase the octane number by at least 5.0 compared to the composition consisting of di-GTBEs and tri-GTBE. The concentration of the mono-GTBEs may be sufficient to increase the octane number between 0.1 and 7.5 compared to the composition consisting of di-GTBEs and tri-GTBE. The concentration of the mono-GTBEs may be sufficient to increase the octane number by at least 0.15 or at least 0.2 compared to the composition consisting of di-GTBEs and tri-GTBE.

Also disclosed herein is an octane boosting composition comprising mono-GTBEs, di-GTBEs and tri-GTBE wherein a concentration of the mono-GTBEs may be above 10 wt% on a total mass basis of all GTBEs.

The concentration of the mono-GTBEs may be above 15 wt%, 20 wt%, 25 wt%, 30 wt%, 40 wt%, 60 wt%, or 80 wt%.

The composition may further include trimethylpentane, and may also include other compounds.

Also disclosed herein is a method of producing Glycerol Tert-Butyl Ethers (GTBE) blends for use in different fuels, comprising: reacting glycerol and isobutene to produce a GTBE blend comprising mono-GTBEs, di-GTBEs and tri-GTBE according to equilibrium reactions; selectively shifting the equilibrium reactions to favour production of a predetermined GTBE blend, comprising: shifting the equilibrium reactions to favour production mono-GTBEs over di-GTBEs and tri-GTBE, to produce a mono-shifted GTBE blend suitable for use in as an octane booster, and shifting the equilibrium reactions to favour production of di-GTBEs and/or tri-GTBE, to produce a di-shifted and/or tri-shifted GTBE blend suitable for use in biodiesel.

Subsequently, the GTBE blend can be optionally increased in mono-GTBE, and/or di-GTBE and/or tri-GTBE content, by the use of a suitable separation step.

The shifting may be performed by changing molar ratios of glycerol and isobutene reactants, changing residence time of the glycerol and isobutene, and/or changing a reaction temperature.

Shifting the equilibrium reactions to produce the mono-shifted GTBE blend may comprise at least one of the steps recited above or herein.

The method may further include separating the mono-shifted GTBE blend in order to increase mono-GTBE content and/or decrease content of di-GTBEs, tri-GTBE, and/or one or more other compounds. The separation can include vacuum based methods and/or extraction based methods.

The invention provides an installation for production of multiple Glycerol Tert-Butyl Ethers (GTBE) blends, comprising: a reaction vessel comprising :a reaction chamber for accommodating the production of GTBE, at least one reactant inlet in fluid communication with the reaction chamber for supplying glycerol and isobutene therein, and a product outlet in fluid communication with the reaction chamber for withdrawing the GTBE blend; and a regulator configured for regulating conditions of the reaction vessel in alternating fashion between a first mode and a second mode, the first mode causing production of a mono-shifted GTBE blend and the second mode causing production of a di- and/or tri-shifted GTBE blend, wherein the regulator is configured to modify a residence time of reactants in the reaction chamber, and wherein the regulator is coupled to the product outlet to regulate withdrawal of the GTBE blend.

Optionally, there can be a GTBE separation unit to optionally increase the mono-GTBE content, or the di-GTBE and/or the tri-GTBE content. The GTBE separation unit can include a distillation unit (e.g., vacuum distillation unit), an extraction unit, and/or another type of separation unit.

In some implementations, the mono-shifted GTBE blend is suitable for use as an octane booster.

In some implementations, the di- and/or tri-shifted GTBE blend is suitable for use in biodiesel.

In some implementations, the regulator is configured to modify molar ratios of the glycerol and the isobutene supplied to the reaction chamber.

In some implementations, the regulator is coupled to the at least one reactant inlet to regulate the molar ratios.

In some implementations, the regulator is configured to modify a reaction temperature in the reaction chamber.

In some implementations, the regulator is further configured for regulating conditions of the reaction vessel in a third mode.

In some implementations, the installation includes a first storage vessel and a second storage vessel coupled to the product outlet of the reaction vessel, and flow direction system adapted to control flow of the GTBE blend into the first or second storage vessel respectively in the first and second modes.

In some implementations, the regulator is configured to enable one or more of the steps and/or obtain features as recited above and/or herein.

In some implementations, the content of the mono-shifted GTBE blend, or the content of the di-shifted GTBE and/or tri-shifted GTBE blend can be further increased by the use of a suitable separation step, e.g., via extraction or vacuum distillation.

In some implementations, the content of mono-GTBE, di-GTBE or tri-GTBE can be shifted in such a manner that a GTBE blend with specific density and/or viscosity is obtained. The viscosity and/or density properties of the GTBEs can be leveraged in various ways in order to enhance mixing, GTBE blend production and performance. Production of GTBE blends can be tailored to a particular fuel to which it is added in order to have viscosity, density, octane-boosting and/or other properties of the blend that have an impact on the fuel's properties (e.g., boosting of octane and increase in density with a mono-shifted blend). Properties such as viscosity and density can be provided so that the GTBE blend has enhanced producibility, post-production mixing with other additives or the fuel, and/or effects on the fuel after mixing therewith, which may be in addition to other advantageous effects such as octane-boosting (e.g., for gasoline) and other effects (e.g., for biodiesel). The composition of the GTBE blend can be formulated and/or generated via equilibrium shifting/control, so that the end product has multiple functionalities that are balanced for particular applications.

### BRIEF DESCRIPTION OF DRAWINGS

Fig 1 is an image of the production of GTBE products from glycerol and isobutene.
Fig 2 is a table illustrating test data regarding octane boosting of GTBE products.
Fig 3 is an image of the side-product spectrum in the synthesis of GTBE^{®} from glycerol and isobutene.
Figure 4 is a schematic scheme of a GTBE production set-up.
Figures 5 and 6 are tables showing experimental results.
Figure 7 is another schematic scheme of a GTBE production set-up.
Figure 8 is a graph of Conversion of IB for experiments performed with varying molar ratio of IB : glycerol and at varying temperatures. The source of isobutene was 50 vol%/50 vol% IB/1-butene.
Figure 9 is a graph of Conversion of glycerol for experiments performed with varying molar ratio of IB : glycerol and at varying temperatures. The source of isobutene was 50 vol%/50 vol% IB/1-butene.
Figure 10 is a graph of Yield of mono-GTBE for experiments performed with varying molar ratio of IB : glycerol and at varying temperatures. The source of isobutene was 50 vol%/50 vol% IB/1-butene.
Figure 11 is a graph of Yield of di-GTBE for experiments performed with varying molar ratio of IB : glycerol and at varying temperatures. The source of isobutene was 50 vol%/50 vol% IB/1-butene.
Figure 12 is a graph of Yield of tri-GTBE for experiments performed with varying molar ratio of IB : glycerol and at varying temperatures. The source of isobutene was 50 vol%/50 vol% IB/1-butene.
Figure 13 is a schematic of an experimental setup used for kinetic and equilibria investigations of a GTBE system.
Figure 14 is a schematic scheme of an experimental setup used for equilibria investigations of a GTBE system.
Figure 15 is a graph of the concentration profile obtained for the reaction of pure mono-GTBE with H₂SO₄ at a temperature of 50 °C.
Figure 16 is a table showing the experimental results of the EQ compositions of pure mono-GTBE with sulphuric acid at varying temperatures.
Figure 17 is a table showing the experimental results of the EQ compositions of pure di-GTBE with sulphuric acid at varying temperatures.
Figure 18 is a graph showing the equilibrium conversions of isobutene and glycerol in the production of GTBE from pure isobutene and pure glycerol as a function of reactant ratio at a temperature of 50 °C.
Figure 19 is a graph showing the equilibrium conversions of isobutene and glycerol in the production of GTBE from pure isobutene and pure glycerol as a function of reactant ratio at a temperature of 80 °C.
Figure 20 is a graph showing the equilibrium conversions of tert-butyl alcohol and glycerol in the production of GTBE from pure tert-butyl alcohol and pure glycerol as a function of reactant ratio at a temperature of 80 °C.
Figure 21 is a graph showing selectivities of isobutene to products in the production of GTBE from pure isobutene and pure glycerol as a function of reactant ratio at a temperature of 50 °C.
Figure 22 is a graph showing selectivities of isobutene to products in the production of GTBE from pure isobutene and pure glycerol as a function of reactant ratio at a temperature of 80 °C.
Figure 23 is a table illustrating test data regarding octane boosting of pure mono-GTBE and pure di-GTBE at varying concentrations.
Figure 24 is a graph of PT-curves of pure mono-GTBE, pure di-GTBE and pure tri-GTBE
Figure 25 is a table showing compositions of the bottom sample and top fractions of a mono-GTBE vacuum distillation run.
Figure 26 is a table showing compositions of the bottom sample and top fractions of a di-GTBE vacuum distillation run.
Figure 27 is a table showing compositions of the bottom sample and top fractions of a tri-GTBE vacuum distillation run.
Figure 28 is a graph showing the densities of mono-GTBE, di-GTBE and tri-GTBE as a function of temperature.
Figure 29 is a graph showing the dynamic viscosities of mono-GTBE, di-GTBE and tri-GTBE as a function of temperature.

### DETAILED DESCRIPTION

A GTBE-based octane booster composition may be produced to have increased mono-GTBE content, and such mono-shifted GTBE blend can be useful for octane boosting particularly due to the enhanced octane boosting effects of mono-GTBE compared to di- and tri-GTBE. Methods and systems for producing a mono-shifted GTBE blend are also disclosed. In addition, an installation is provided for selectively producing different GTBE blends for different uses.

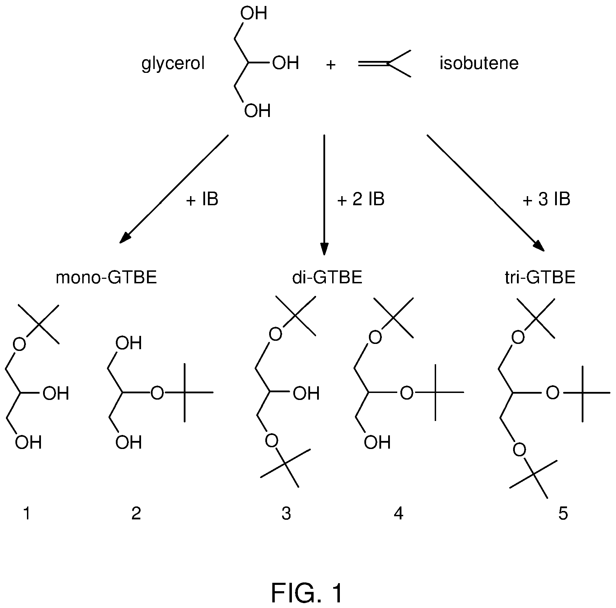

GTBE is a mixture of tert-butyl ethers of glycerol and there are five possible GTBE molecules: two isomers of mono-GTBE; two isomers of di-GTBE, and tri-GTBE. The production of GTBE includes several equilibrium reaction steps, shown in Figure 1. Glycerol and isobutene are used as reactants. A homogeneous or heterogeneous catalyst can be used to enhance the reaction rate.

The IUPAC names of the products illustrated in Figure 1 are (1) 3-tert-Butoxypropane-1,2-diol; (2) 2-tert-Butoxypropane-1,3-diol; (3) 1,3-di-tert-Buoxypropane-2-ol; (4) 2,3-di-tert-Butoxypropane-1-ol; and (5) 1,2,3-di-tert-Butoxyropane. Products (1) and (2) will be referred to herein as mono-GTBE, products (3) and (4) will be referred to herein as di-GTBE and product (5) will be referred to herein as tri-GTBE.

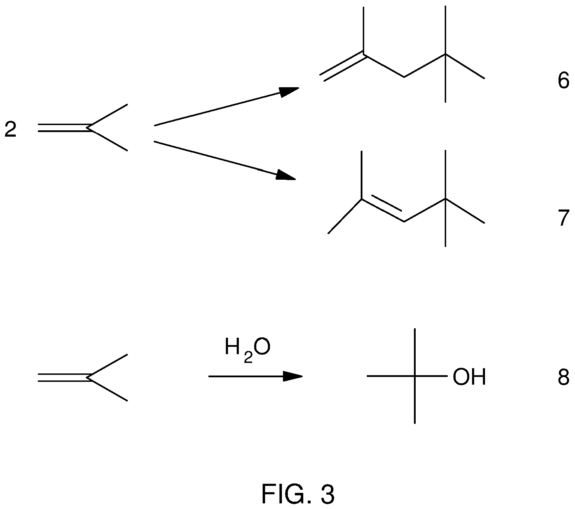

It should also be noted that undesired oligomerization and hydration of isobutene can occur. Such reactions are shown in Figure 3. The IUPAC names for the side-products are (6) 2,4,4-Trimethyl-1-pentene; (7) 2,4,4-Trimethyl-2-pentene; and (8) 2-Methyl-2-propanol (or tert-butanol). Products (6) and (7) will be referred to as TMP and product (8) will be referred to as TBA.

It is also noted that tert-butyl alcohol can react with glycerol to form GTBE. In addition, gas streams containing isobutene (e.g., raffinate-I) can be used as a reactant. Compounds can react to form isobutene for the production of GTBE (e.g., tert-butyl alcohol or isobutanol dehydration).

Glycerol and isobutene may be contacted in a reaction vessel under conditions to generate a blend of Glycerol Tert-Butyl Ethers (GTBE) comprising mono-GTBEs, di-GTBEs and tri-GTBE. The reaction vessel conditions and the molar ratios of the glycerol and isobutene reactants can be regulated to favor production of mono-GTBEs according to equilibrium reactions, thereby producing a mono-shifted GTBE blend. The mono-shifted GTBE blend can be used with gasoline as an octane booster, and due to the higher mono-GTBE content the octane boosting is enhanced.

Referring to Fig 1, the amount of isobutene required to produce GTBE is highest for tri-GTBE. As glycerol is the cheapest reactant, and isobutene is the most expensive reactant, it is additionally advantageous to produce and use mono-GTBE as a product because production costs can be reduced compared to the counterpart di- and tri-GTBE. Glycerol can be obtained economically due to overproduction in the biodiesel industry, for example. Thus, mono-shifted GTBE blends can not only increase octane boosting effects but can also be produced more economically and using less isobutene reactant.

The GTBE production reactions are equilibrium reactions. Investigations were conducted to assess manipulation of the process to shift production toward certain types of GTBE (e.g., transform di-GTBEs into mono-GTBEs or impact proportion of products based on molar ratios of reactants entering the reactor vessel), and to assess to what extent pure GTBE compounds can be converted to other GTBE compounds. Studies revealed that it is possible to manipulate the GTBE type during production, and shift the proportions of the GTBEs being produced and convert pure GTBE compounds to other GTBE compounds. Moreover, varying the molar ratio of the reactants influences reactor composition. For example, if mono-GTBE is desired in a specific time, the process can be manipulated to shift toward increased production of mono-GTBEs. Likewise, if a greater proportion of di-GTBE is desired, the process can be manipulated to optimize that type of GTBE being produced. Based on the conducted experimental work a thermodynamic model was constructed to predict equilibrium compositions at varying settings, e.g., varying temperatures and varying reactant ratios. Subsequent to the production step, a separation step could be included to further purify mono-GTBE, or di-GTBE, or tri-GTBE, for example by extraction or (vacuum) distillation.

Experiments were conducted to investigate the influence of temperature, isobutene to glycerol molar ratio, and the source of isobutene on the production of GTBE. Additional experiments were performed to assess the extent to which pure GTBE compounds can be converted to other GTBE compounds. With respect to purification, studies were performed to determine the PT-curves of the varying pure GTBEs, which were obtained after purification via vacuum distillation.

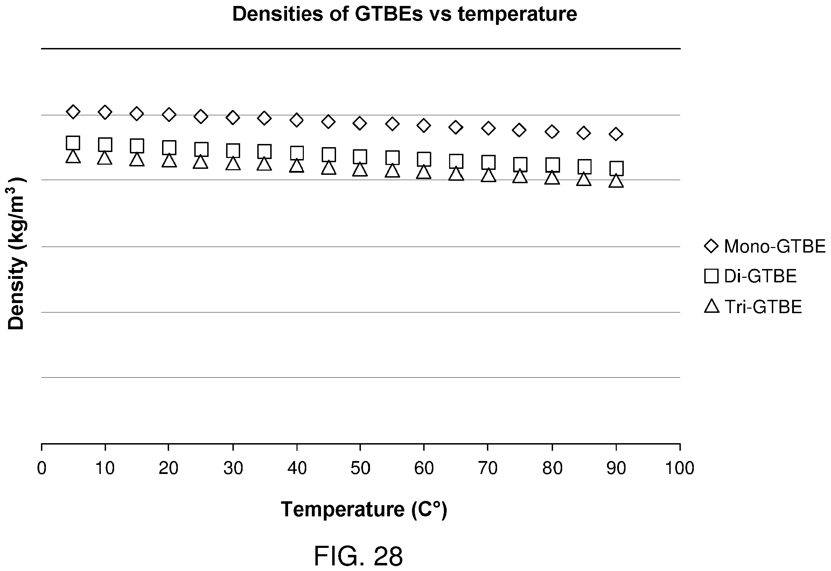

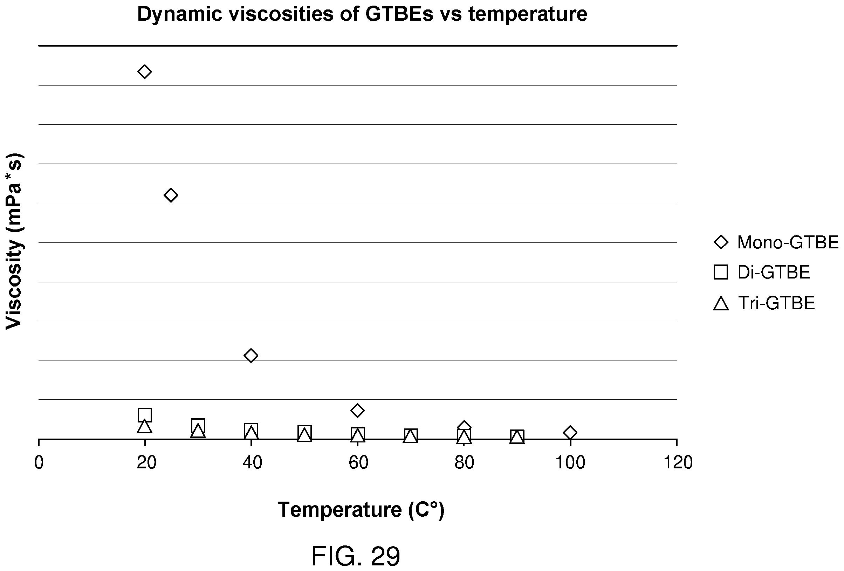

GTBE blend with specific characteristics with respect to density and dynamic viscosity could be desirable. Experiments were conducted to determine the difference in density and dynamic viscosity among the varying types of GTBE, and determine the effect of temperature on both the densities and dynamic viscosities of the different GTBEs (i.e. pure mono-GTBE, pure di-GTBE, and pure tri-GTBE). Results are shown in Experimentation 9 below and Figures 28 and 29.

Manipulating viscosity an/or density properties can be performed in concert with the particular composition of the GTBE blend and its end use (e.g., in gasoline). Such manipulations can be done during the production of GTBE blend to obtain a blend having target viscosity and/or density properties, or after the GTBE blend is formed in the context of mixing the GTBE blend with an end-use product. For example, when a GTBE blend having a target viscosity and/or density properties (e.g., target ranges of viscosity and/or density) is desired, the process conditions can be regulated to obtain certain amounts or proportions of mono-, di-, and tri-GTBE such that the aggregate properties are within the target range. For instance, if a GTBE blend with as much mono-GTBE as possible while staying below a target threshold viscosity value is desired, the process conditions can be operated to shift the reaction equilibria to produce the GTBE blend having a certain amount of mono-GTBE which have high viscosity and would thus increase the aggregate viscosity of the blend. If particular density or viscosity profiles are desired for the GTBE blend, the process conditions can be regulated to shift the reaction equilibria in order to produce the GTBE blend with the target properties.

Another example of leveraging GTBE viscosity manipulation is by increasing the temperature of the GTBE blend based on its composition (e.g., based on its mono-GTBE content which causes higher viscosity) for enhanced mixing with an end-product hydrocarbon, such as gasoline or biodiesel. Mixing of liquids (e.g., GTBE blend and gasoline) can be enhanced when the two liquids have similar viscosities. For a given GTBE blend with a high mono-GTBE content, the mixing temperature of the GTBE blend can be increased in accordance with the high mono-GTBE content to reduce the viscosity down below a target level (e.g., similar to that of the liquid into which the GTBE will be mixed). Figure 29 shows that mono-GTBE has high viscosity at temperatures below 30°C or 20°C, but as the temperature is increased the viscosity dramatically decreases. Thus, for high mono-GTBE blends, higher mixing temperatures can provide enhanced production of the octane boosted fuel.

Yet another way to leverage GTBE viscosity manipulation is in the context of adding a particular GTBE compound (e.g., mono-GTBE) to a GTBE blend in order to increase the amount of that GTBE compound in the blend. By controlling the temperature of the GTBE blend and/or the added GTBE compound, similar viscosities can be achieved between the two liquids for enhanced mixing. For example, if mono-GTBE is to be added to a blend, then it can be pre-heated to a temperature at which the mono-GTBE blend has a similar or identical viscosity to the GTBE blend or is within a certain percentage (e.g., 50%, 40%, 30%, 20%, or 10%). For instance, the mono-GTBE can be preheated to at least 50°C, 60°C, 70°C, 80°C or 90°C so as to decrease its viscosity during mixing with a blend. When the blend has a certain mono-GTBE content, the blend can also be preheated to decreases its viscosity. In scenarios where the blend has a high mono-GTBE content and mono-GTBE is being added to it to further increase the mono-GTBE concentration, both the blend and the added mono-GTBE can be heated to temperatures (which may be the same or different) to provide similar or generally identical viscosities for mixing.

Furthermore, the viscosity and/or density properties of the GTBE blend can be tailored or predetermined such that the GTBE blend has properties that will have a desired impact on the fuel into which it is added. For example, the production process can be performed under conditions to shift the equilibrium reactions to produce a GTBE blend that has a certain composition of mono-, di- and tri-GTBE with higher mono-GTBE content and corresponding higher density characteristics so that the GTBE blend can increase the density of the fuel to which it is added. Thus, the GTBE blend can be provided with viscosity and/or density properties that will increase or decrease the viscosity and/or density of the fuel (e.g., gasoline, biodiesel, etc.) to which it is added, so that the GTBE blend can have multiple functionalities (e.g., octane booster and density booster).

It is also noted that mixing can be provided in the reaction vessel in which GTBE blends are produced, particularly when raffinate-I is the source of isobutene as two liquid phases will remain in the reactor throughout the GTBE production run.

### Experimentation 1

The influence of the molar ratio of isobutene to glycerol, as well as the temperature, were investigated on the production of GTBE. Pure isobutene and pure glycerol were used as reactants.

### Description of setup

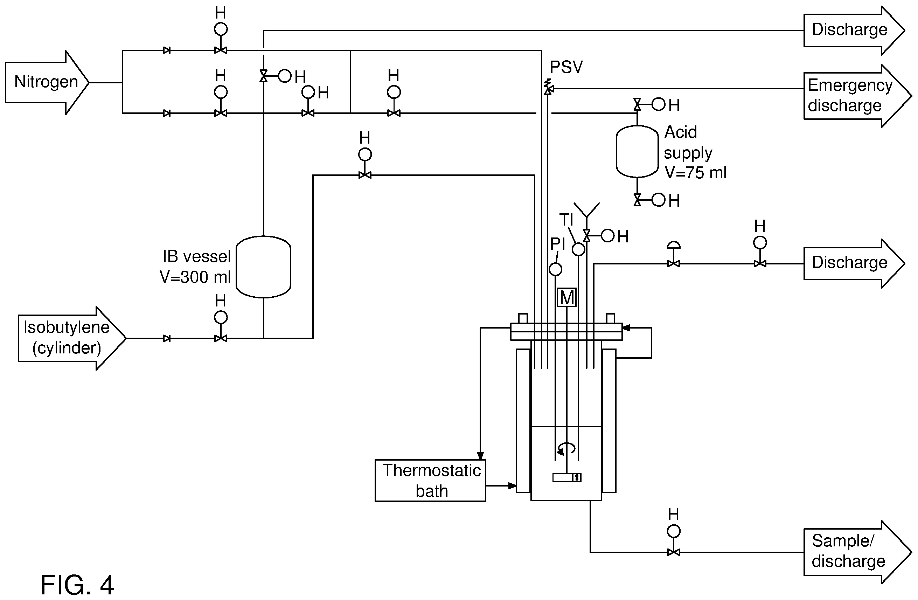

The GTBE setup included a batch wise operated stirred tank reactor with a total volume of 8 litres. The reactor was equipped with baffles, a jacket, a pressure gauge, a temperature indicator, a drain valve, a funnel, an isobutene dosing system, an acid dosing system and a pressure relief valve. The isobutene dosing system included an isobutene gas bottle, a 300 ml gas bomb and interconnecting tubing with manual operated valves. The reactor was heated with aid of a thermostatic bath with temperature control. The stirring speed could be manipulated with aid of a frequency converter. Each experiment continued until a significant drop in pressure was notified (several bars). The sulphuric acid content was 3 wt% of glycerol content. At the end of each experiment a liquid sample was taken for analysis. Figure 4 shows a schematic scheme of the GTBE set-up.

### Results

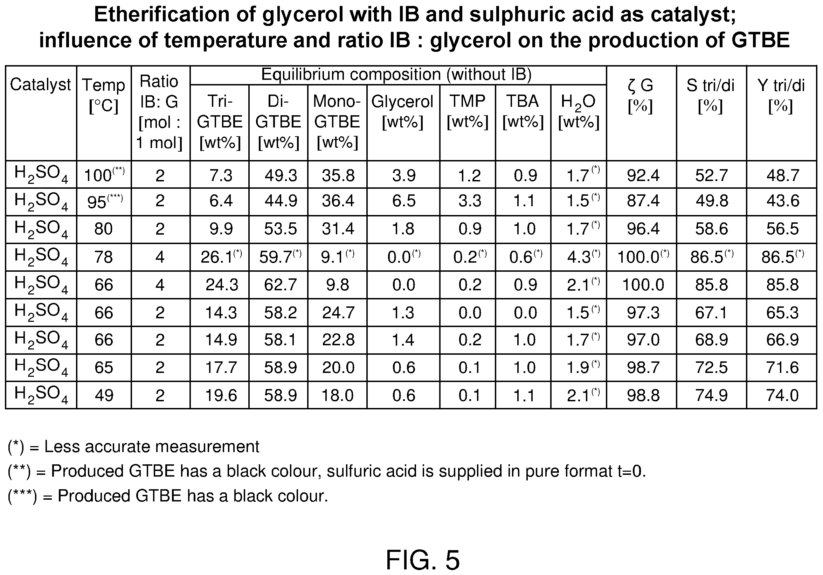

Table 1 illustrated as Figure 5 shows the results of several IB to GTBE production experiments. From Table 1 the following conclusions were drawn:
(i) Above a reaction temperature of 80°C the undesired formation of the by-product TMP increases to above 1 wt%.
(ii) Increasing the molar ratio of IB to glycerol from 2 :1 to 4 :1 the content of tri-GTBE increases 10 - 15 wt%, the content of di-GTBE increases 4 - 6 wt% and the content of mono-GTBE decreases 15 - 22 wt%. In general it can be stated that the increase in molar ratio of IB to glycerol increases the yield of tri-GTBE and lowers the yield of mono-GTBE. Also the conversion of IB is lowered (not shown).
(iii) The glycerol conversion equals 97 - 100% for a molar ratio of IB to glycerol of 2 : 1 at a reaction temperature of 65 °C. The tri- and di-GTBE selectivity respectively yield 67 - 72% and 65 - 72%. Increasing the temperature from 49°C to 100 °C decreases the selectivity towards di- and tri-ethers for a molar ratio of IB to glycerol of 2 : 1.

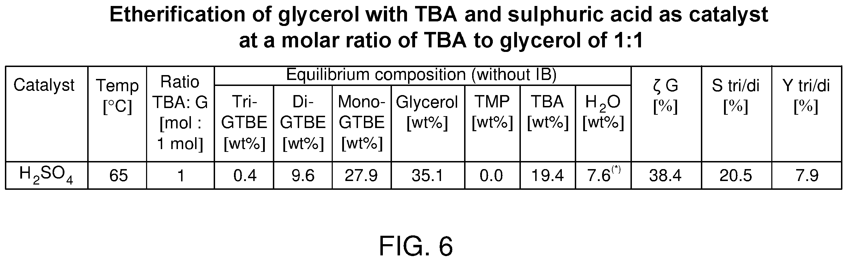

Table 2 illustrated as Figure 6 shows the results of a TBA to GTBE production experiment. From Table 2 it can be concluded that performing the etherification with TBA instead of IB lowers the glycerol conversion and selectivity and yield of di- and tri-GTBE.

### Experimentation 2

The influence of the molar ratio of isobutene to glycerol, as well as the temperature, were investigated on the production of GTBE. A mixture of 50 vol%/50 vol% isobutene/1-butene and pure glycerol were used as reactants.

### Description of setup

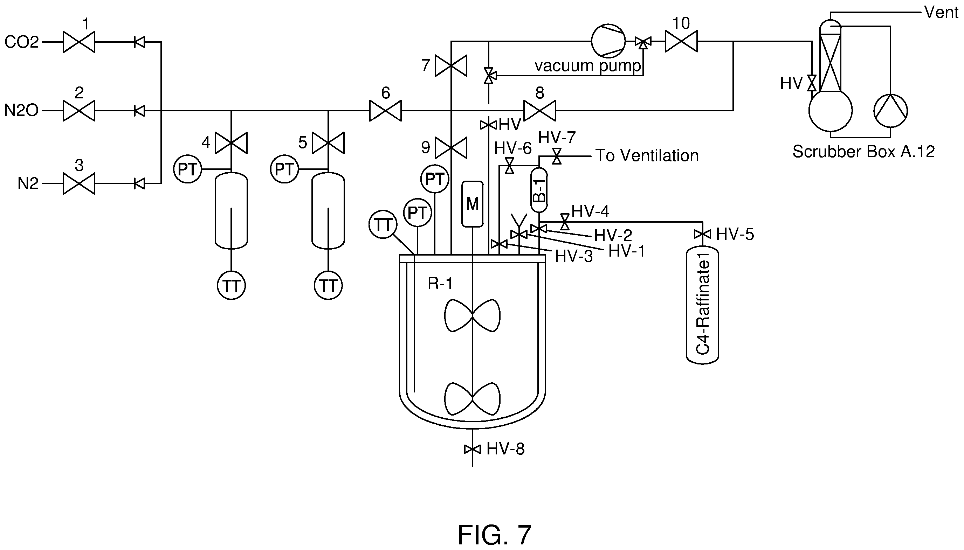

Experiments were performed in a 1 litre stainless steel autoclave. The autoclave could handle pressures up to 60 bar and temperatures up to 250 °C. The reactor was equipped with glass windows. The reactor was operated batchwise. A vacuum pump was used to evacuate air in the reactor. Nitrogen could be fed to the reactor. The reactor temperature was regulated with a Julabo heater. A blade stirrer was operated with a magnetic drive. The blades were bent to enhance mixing of the two liquid phases and ensure sufficient interfacial area. Stirring speeds up to 1800 rpm could be achieved. Baffles inside the reactor promoted mixing. The temperature and pressure were monitored during each experiment. A gas bomb of 100 ml was used to enter isobutene to the reactor. Sampling was performed via a sampling valve at the bottom of the reactor. Samples were analysed by gas chromatography. Figure 7 shows a schematic representation of the experimental setup.

### Results

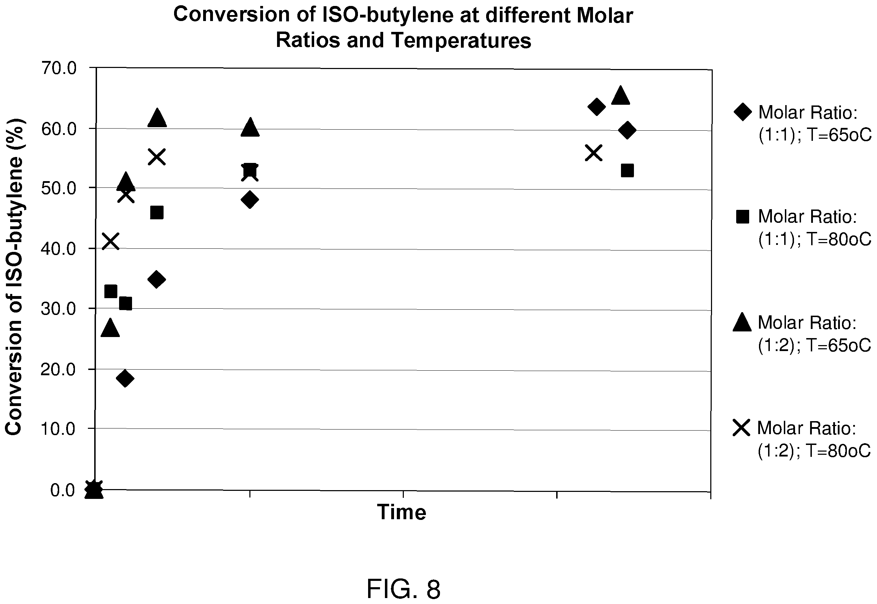

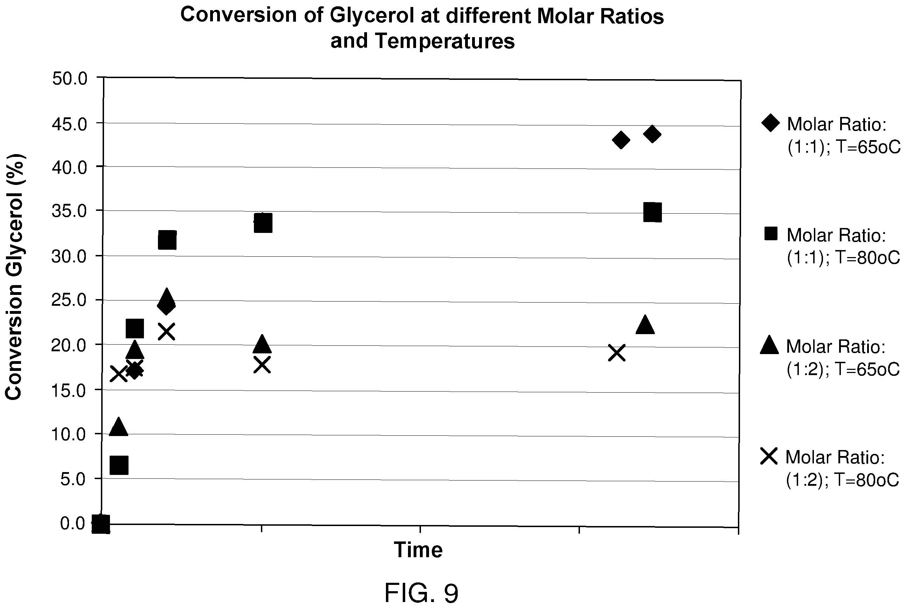

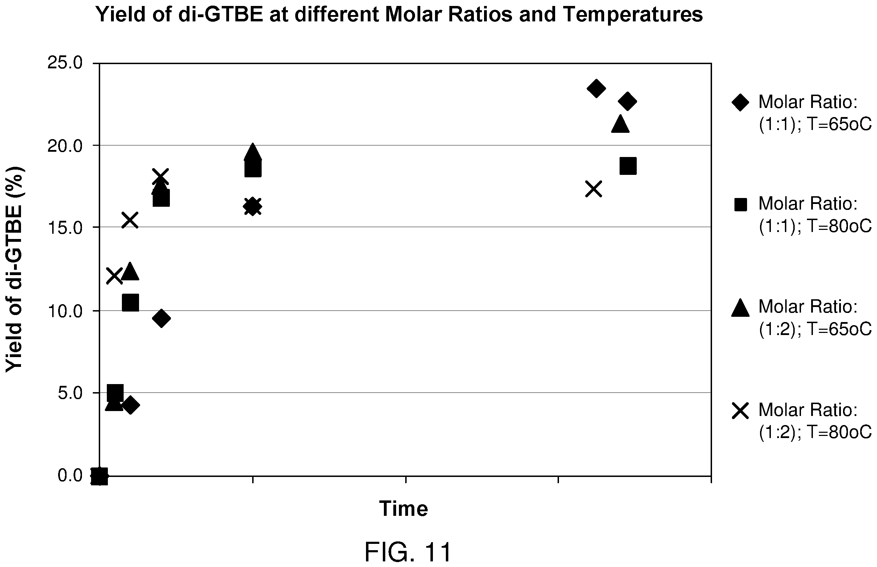

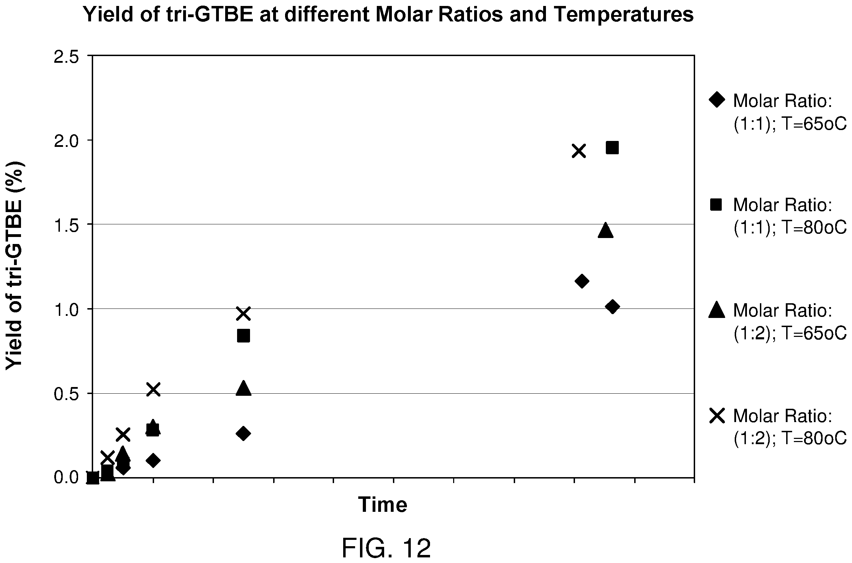

Figures 8 to 12 show the results of several 50 vol% / 50 vol% isobutene/1-butene to GTBE production experiments.

For experiments performed with pure glycerol, conversions of glycerol were in the range of 20% to 45%. The highest glycerol conversion of 45% was accomplished with the experiment with an isobutene to glycerol molar ratio of 1:1 at 65 °C. Lower conversions of glycerol were obtained at a molar ratio of 1:2. The isobutene conversion profiles follow the same trend as the total GTBE yield profiles. For experiments performed with pure glycerol, the conversion of isobutene is in the range of 55% to 65%. The highest yield of GTBE, i.e. mono-, di- and tri-GTBE, was observed for the experiment performed at 65°C with an isobutene to glycerol molar ratio of 1:2.

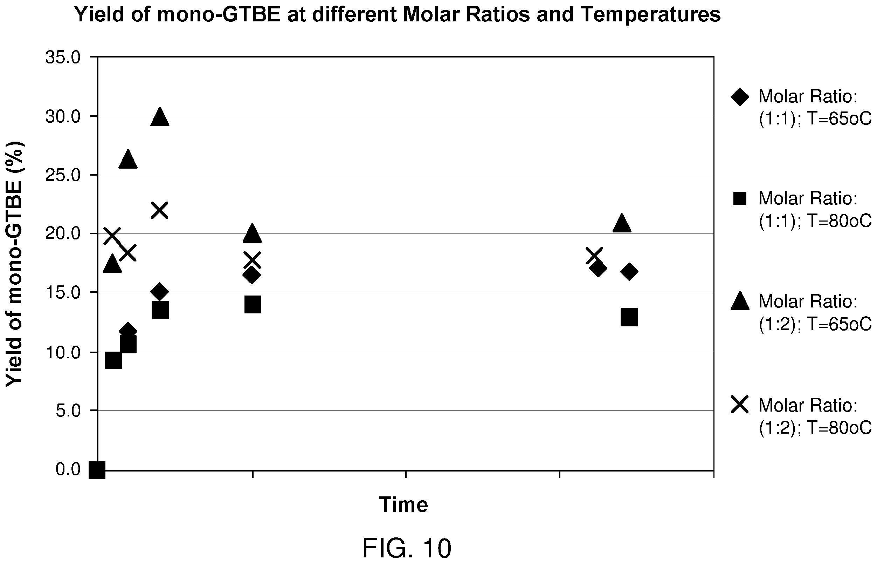

Interestingly, the mono-GTBE yield can be influenced via the residence time in the reactor, as depicted by Figure 10. Therefore, when a high yield of mono-GTBE is desired, both the molar ratio of reactants, as well as the temperature and residence time in the reactor, can be used to steer the production of mono-GTBE. For example, in some scenarios, the residence time can be shortened by about half to yield a corresponding increase in mono-GTBE yield of about 50% (e.g., see Fig 10 data where halving the time resulted in increasing yield from 20% to 30% mono-GTBE at 1 : 2 molar ratio and 65°C). It can be seen that there is a peak zone of mono-GTBE yield at lower residence times, where the yield is above 25% (at 65°C) and is above 20% (at 80°C). Operating the production process within such a peak zone can thus yield higher concentrations of mono-GTBE (e.g., yields above 20%, 22%, 24%, 26%, 28%, or 30%).

### Experimentation 3

Experiments were conducted to assess to which extent pure mono-GTBE and pure di-GTBE could be converted to other GTBE compounds. The influence of temperature and reaction time on the reactor composition were investigated.

### Description of setups

The experiments were performed in a high-pressure autoclave and a low-pressure glass setup for experiments in which there was no pressure build-up.

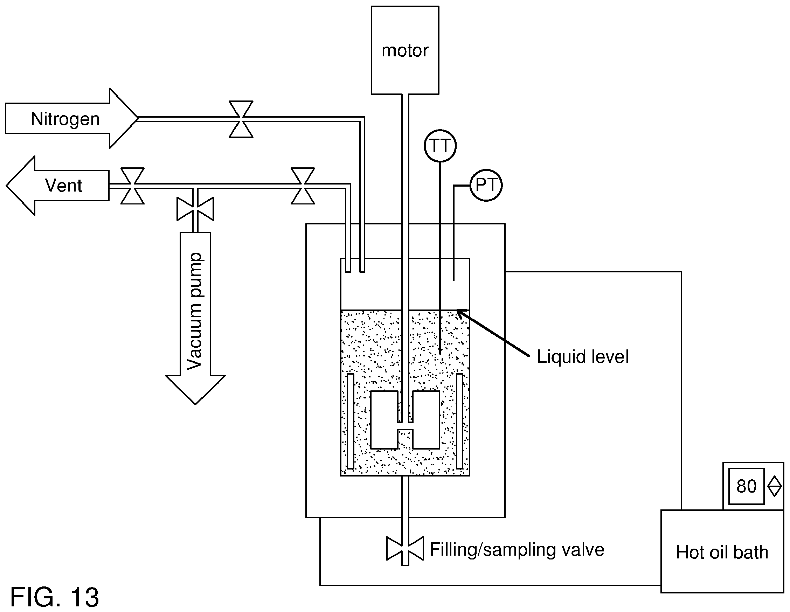

The 0.5 I high-pressure autoclave could handle pressures up to 200 bar and temperatures up to 250°C. The reactor was operated batchwise. A vacuum pump was used to evacuate air in the reactor. Nitrogen could be fed to the reactor. The reactor temperature was regulated with a Julabo heater. A blade stirrer was operated with a magnetic drive. Baffles inside the reactor promoted mixing. The temperature and pressure were monitored during each experiment. Sampling was performed via a sampling valve at the bottom of the reactor. Samples were analysed by gas chromatography. Figure 13 shows a schematic representation of the experimental setup. Due to safety reasons, the stirrer motor and oil bath could not be left turned on overnight. Therefore, stirring and heating could only be performed during daytime.

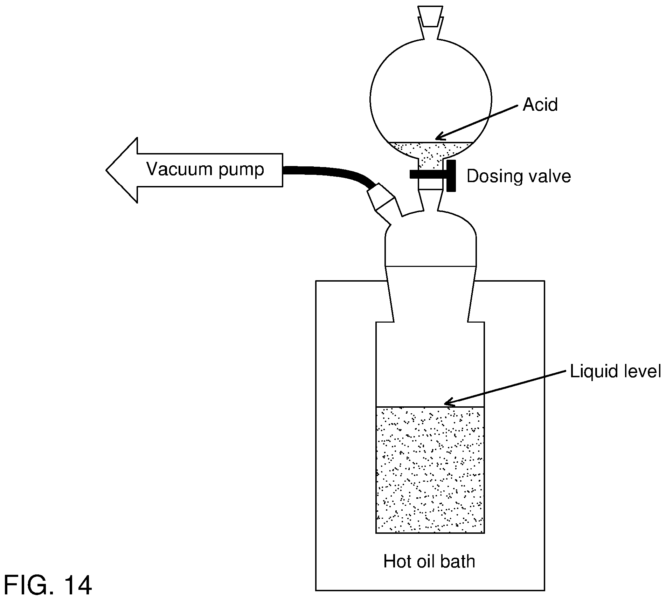

The low-pressure glass setup could be continuously operated for longer periods of time compared to the high-pressure autoclave with smaller amounts of GTBE (approximately 20 ml). The liquid was stirred with a magnetic stirrer. No intermediate sampling was performed, one sample after prolonged period of time provided the equilibrium composition. Figure 14 shows a schematic representation of the experimental setup

### Results

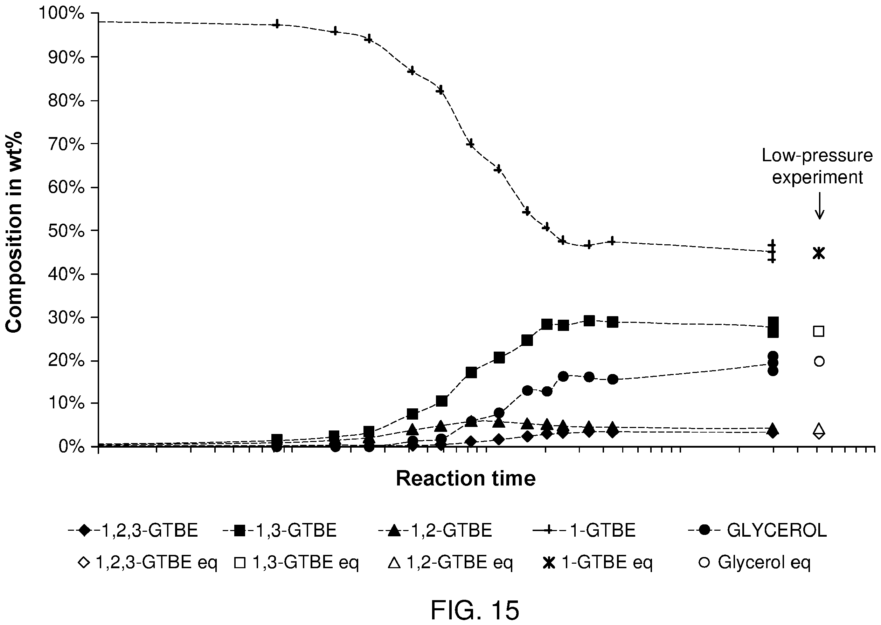

Figure 15 shows the concentration profile obtained for the reaction of mono-GTBE with H₂SO₄ at a temperature of 50 °C (as an example of a pure GTBE with H₂SO₄ reaction). From Figure 15 can be concluded that mono-GTBE is partially converted. The main components present at EQ are mono-GTBE, di-GTBE and glycerol. Both the high-pressure apparatus and the low-pressure apparatus resulted in the same composition at the end of reaction.

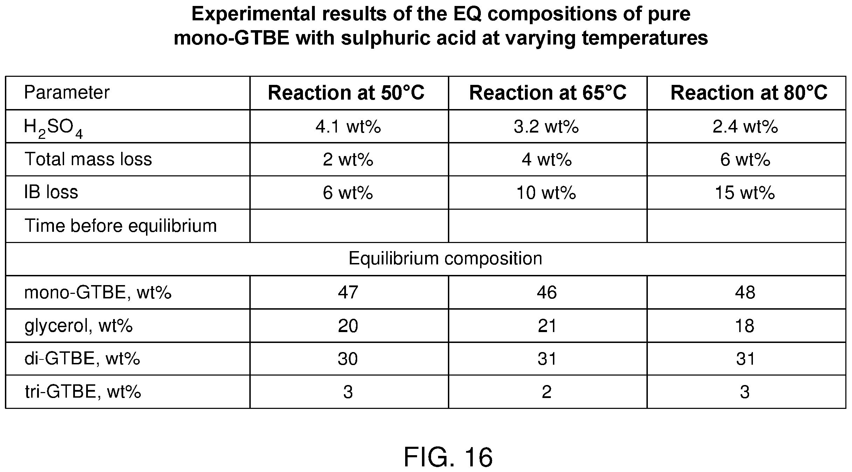

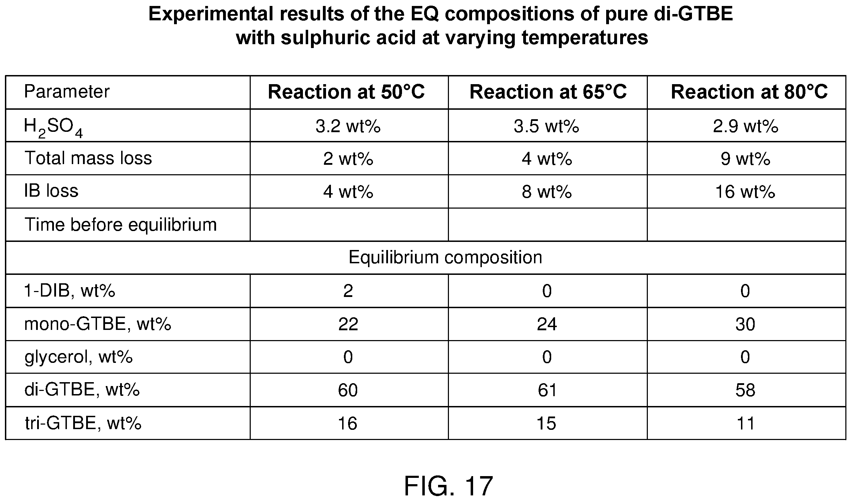

Tables 3 and 4, illustrated as Figures 16 and 17 respectively, show the equilibrium compositions of mono-GTBE with H₂SO₄ and di-GTBE with H₂SO₄ at varying temperatures respectively. From Figure 16 (pure mono-GTBE) can be concluded that the temperature did not significantly affect the equilibrium composition. Only small amounts of tri-GTBE were formed. However, an increase in temperature resulted in an increased formation of isobutene. Unlike experiments performed with pure mono-GTBE, experiments performed with pure di-GTBE (Figure 17) did not result in the formation of glycerol. Di-GTBE was partially converted. The main components at EQ are di-GTBE, mono-GTBE and tri-GTBE.

Figures 15 to 17 indicate that pure mono-GTBE and pure di-GTBE can be converted partially into other GTBE compounds.

### Experimentation 4

A tailor-made numerical program was previously developed to be able to perform thermodynamic model simulations of the production of GTBEs (ref: Wermink, W.N. et al.: "GTBE - Turning residual biodiesel glycerin into a remedy for diesel soot emissions; thermodynamic background," 15th European biomass conference and exhibition; from research to market development, 2007, p 2045 - 2049). A predictive function was implemented to be able to predict equilibrium compositions at varying settings, in particular varying temperatures and varying reactant compositions.

The thermodynamic model was constructed from equilibrium constant temperature relationships of the involved reactions in the production of GTBE from pure isobutene or pure tert-butyl alcohol and pure glycerol. The thermodynamic relationships were derived from experimental work and literature. The GTBE system is non-ideal, because the components present in the reacting system differ in polarity, structure and dimension respectively. The deviation from ideal behaviour was accounted for by introducing activity coefficients. Activity coefficients were calculated with an activity coefficient method based on group contribution theory. The predictive function is a numerical code with which the sets of mole fractions and activity coefficients at equilibrium are determined through iteration. It calculates the optimum solution for which the difference among the theoretical and calculated chemical equilibria of the different reactions occurring in the GTBE system is the smallest.

### Results

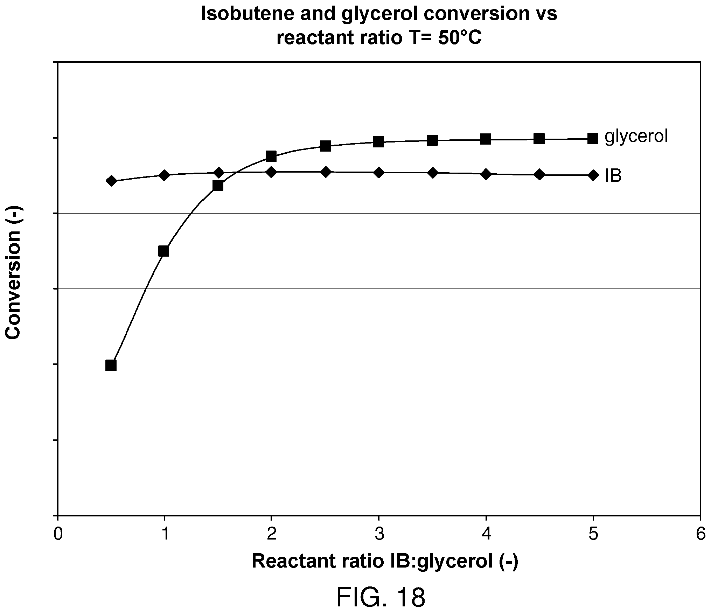

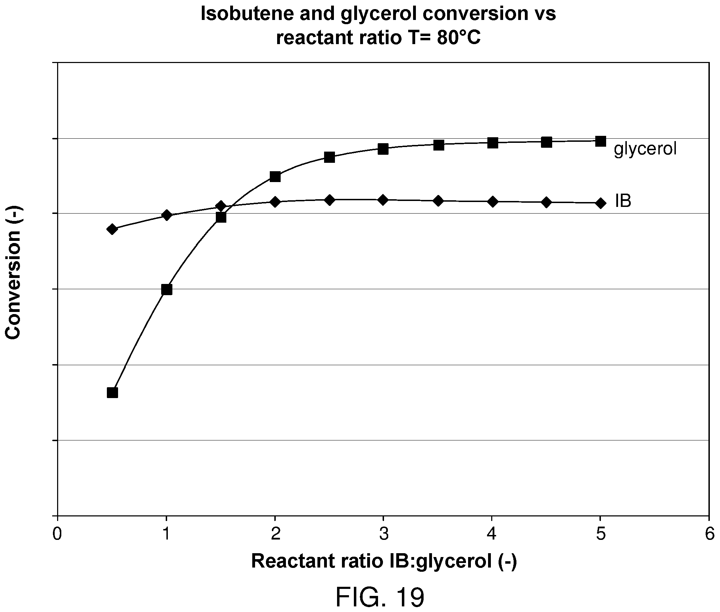

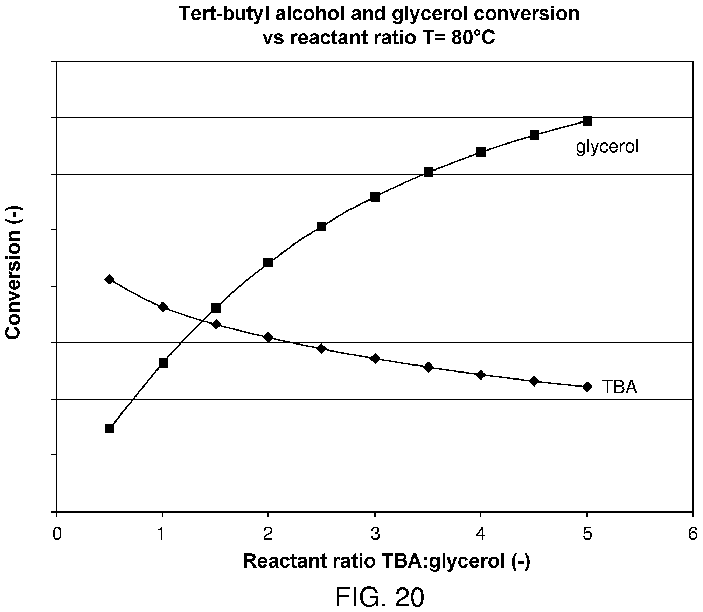

Figures 18 and 19 show the conversions of isobutene and glycerol as a function of the reactant ratio at temperatures of 50 °C and 80 °C respectively. Figure 20 shows the conversions of TBA and glycerol as a function of the reactant ratio at a temperature of 80 °C. An increase in temperature results in a slight decrease in both IB and glycerol conversions. Conversions are significantly lower when GTBE is produced from tert-butyl alcohol compared to isobutene.

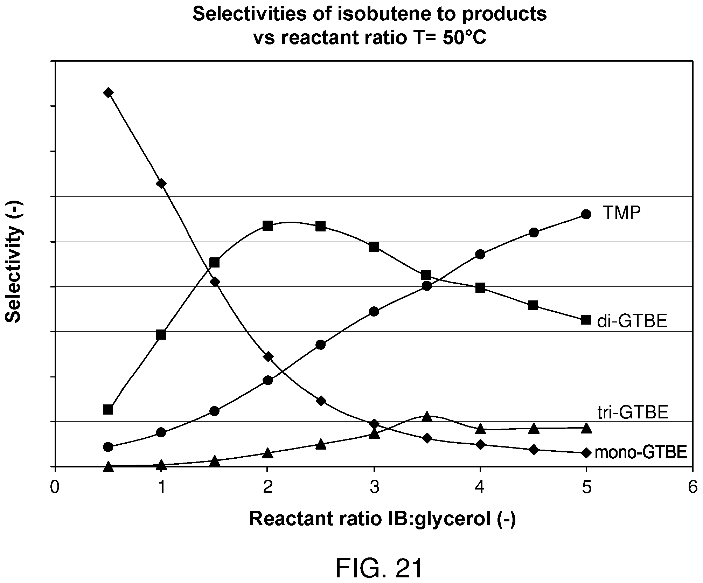

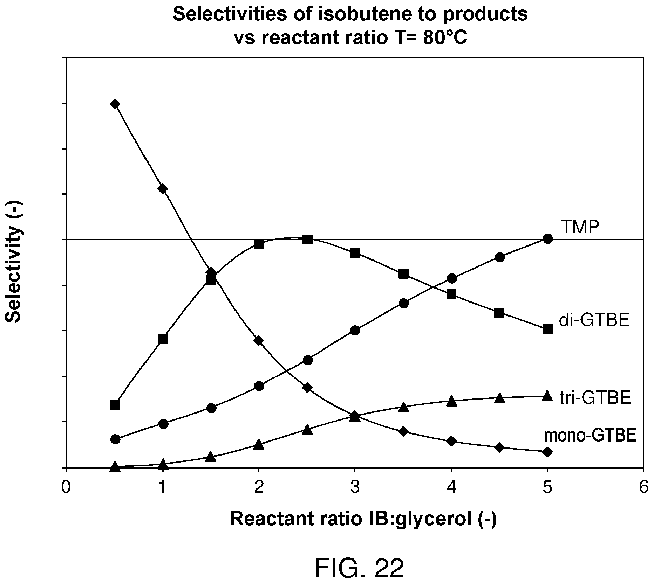

Figures 21 and 22 show selectivities of isobutene to products as a function of the reactant ratio at temperatures of 50 °C and 80 °C respectively. An increase in temperature slightly lowers the selectivity to di-GTBE and slightly increases the selectivity to tri-GTBE. An optimum in di-GTBE selectivity is observed at a reactant ratio of approximately v = 2.2. Mono-GTBE has the highest selectivity till a reactant ratio of around 1.5, di-GTBE has the highest selectivity from a reactant ratio of 1.5 to 3.8 and for reactant ratios higher than 3.8 trimethylpentene has the highest selectivity.

From the results, it can be concluded that a change in reactant ratio significantly influences the equilibrium composition.

### Experimentation 5

Experiments were conducted to assess octane boosting effects of different GTBE types. To do so, a GTBE blend was separated into mono-GTBE, di-GTBE and tri-GTBE, and each was then subjected to octane and other tests. The separation included multiple vacuum distillation runs.

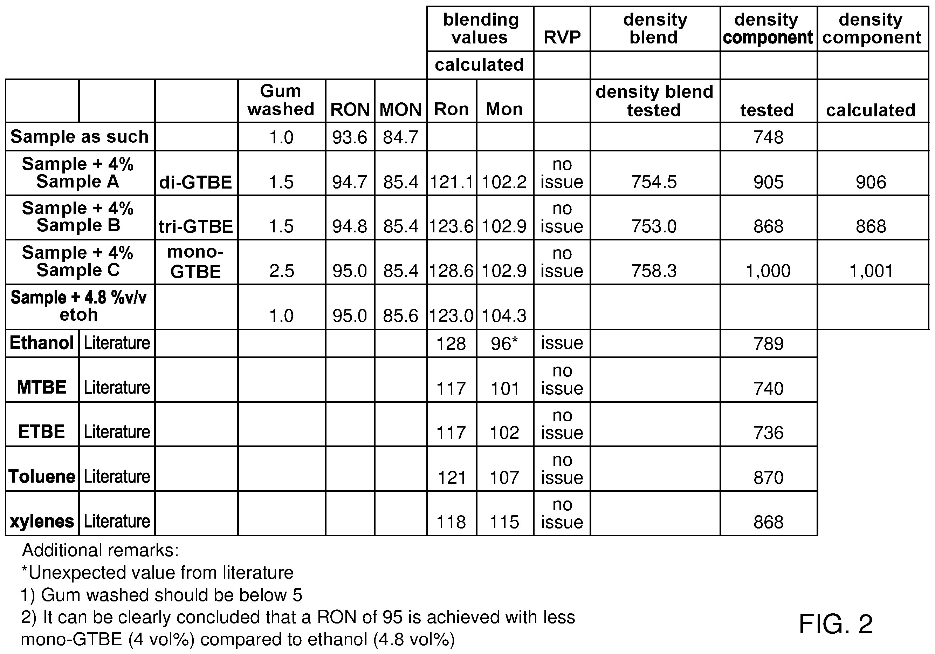

Figure 2 presents results that illustrate effects of the different types of GTBE on the octane number. For example, it was found that mono-GTBE provides the highest octane boosting octane of all of the GTBEs that were tested, particularly the Research Octane Number (RON). The tests to determine density, RON, MON and Gum Content were conducted according to ISO 12185, EN ISO 5164, EN ISO 5163 and EN ISO 6246 respectively.

### Experimentation 6

Additional experiments to assess octane boosting of pure mono-GTBE and pure di-GTBE in fuels containing varying GTBE concentrations were conducted at facilities different from those of Experimentation 4. This investigation was performed a.o. to investigate whether an increase in GTBE content resulted in fuel mixing problems and to determine if an increase in GTBE content results in an increase in octane number measured.

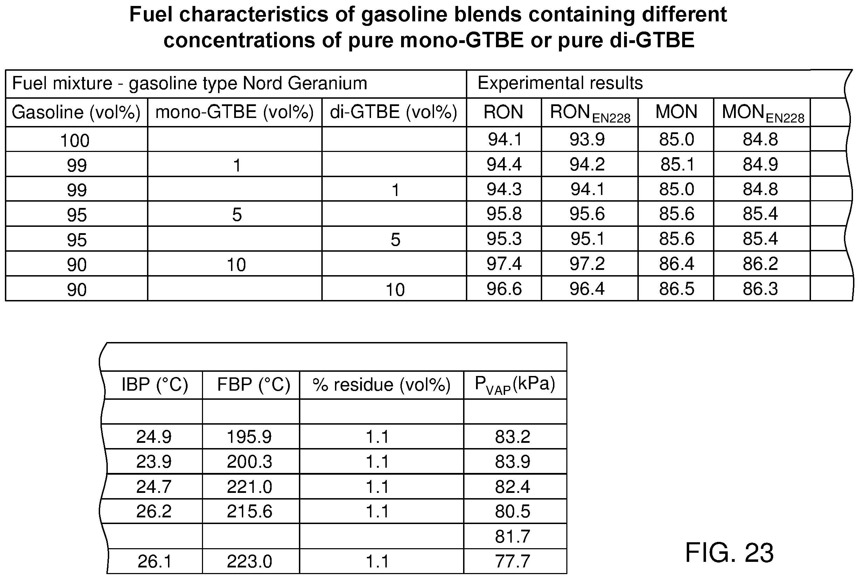

Table 5 illustrated as Figure 23 shows the effects of pure mono-GTBE and pure di-GTBE, added in varying fractions to gasoline, on a.o. octane number and other fuel characteristics. For example, in agreement with Experimentation 5, it was found that mono-GTBE provides the highest boosting of octane of the GTBEs tested. Moreover, an increased content of both mono-GTBE or di-GTBE resulted in an increased boosting of octane. No fuel mixing problems were encountered with blending GTBEs up to a fraction of 10 vol%. An increase in final boiling point was observed with an increased content of pure mono-GTBE or pure di-GTBE. The tests to determine RON, MON, initial boiling point (IBP) and final boiling point (FBP), and vapour pressure (air saturated) were conducted according to the international standards IP 237, IP 236, IP 123 and IP 394 respectively.

### Experimentation 7

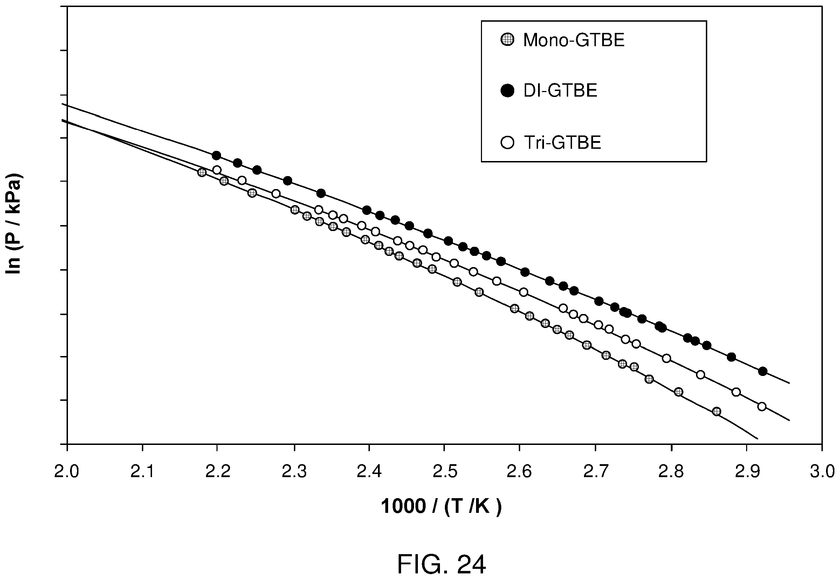

Experiments were performed to determine PT-curves of pure mono-GTBE, pure di-GTBE and pure tri-GTBE. The experimental data of the different GTBEs were fitted to the Clausius Clapeyron equation. Figure 24 presents the PT curves of pure mono-GTBE, pure di-GTBE and pure tri-GTBE respectively. The dots represent the experimental points, the solid line represents the simulated points. For example, it was observed that the vapour pressure of di-GTBE is highest among the different GTBEs, and that the vapour pressure of mono-GTBE is lowest among the different GTBEs.

### Experimentation 8

As explained in Experimentations 1 to 4 a GTBE production run will always result in a mixture of GTBEs; pure GTBE compounds cannot be produced in a single reactor step. Further purification can be performed via for example vacuum distillation.

### Description of setup

The vacuum distillation unit used for GTBE purification and separation was a batch-wise operated vacuum distillation unit consisting of an insulated glass column with a height of 1.5 m and packed with 5 × 5 mm Raschig rings. The setup is equipped with a temperature-controlled heater, a vacuum pump and a Julabo cooler to provide cooling liquid to the top condenser. Bottom and top temperatures, as well as pressure, were monitored. The sample in the bottom flask was stirred during distillation with a magnetic stirrer. A gas wash flask was connected between the vacuum pump and the top condenser to prevent liquids entering the vacuum pump.

### Results

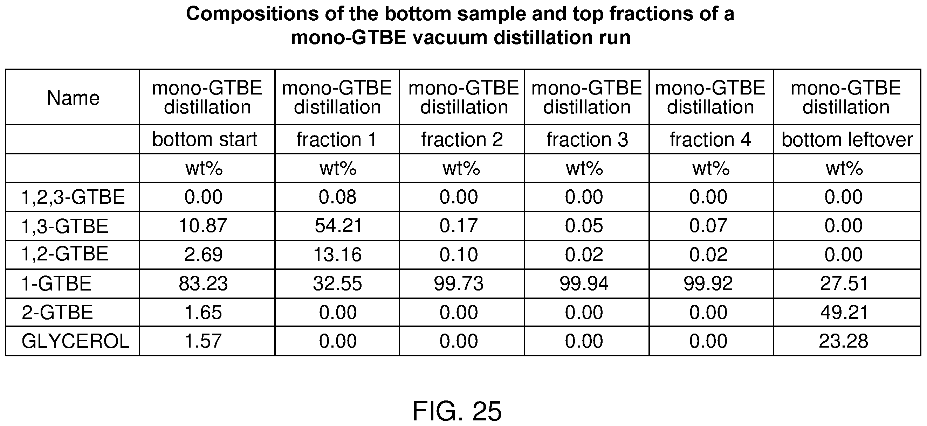

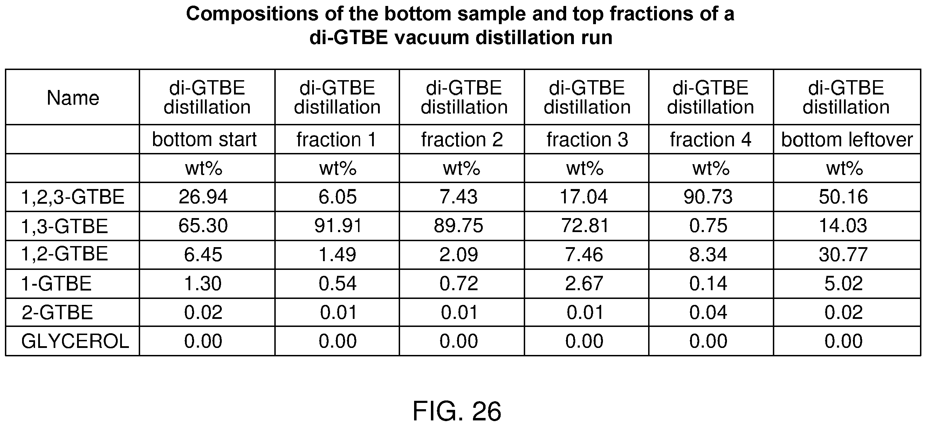

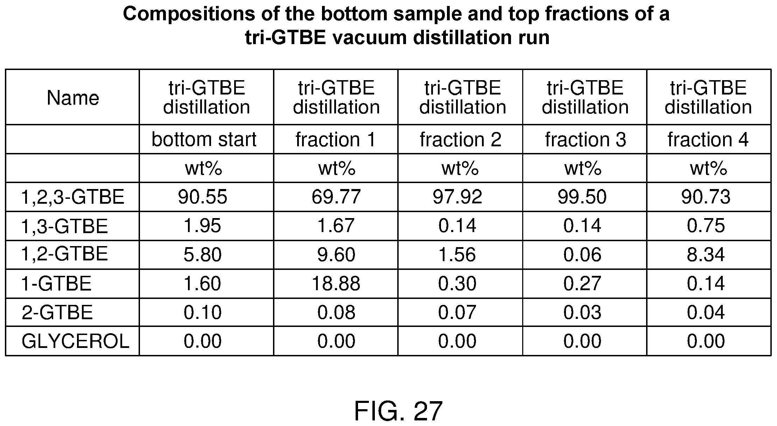

Table 6, illustrated as Figure 25, presents the compositions of the bottom sample, containing a large amount of mono-GTBE, and the top fractions obtained during distillation. Table 7, illustrated as Figure 26, presents the compositions of the bottom sample, containing a large amount of di-GTBE, and the top fractions obtained during distillation. Table 8, illustrated as Figure 27, presents the compositions of the bottom sample, containing a large amount of mono-GTBE, and the top fractions obtained during distillation.

For example, it can be concluded that a mixture of GTBEs can be further purified to pure mono-GTBE, pure di-GTBE or pure tri-GTBE. Moreover, to obtain pure mono-GTBE, or pure di-GTBE, or pure tri-GTBE, several vacuum distillation runs could be required (depending on the amount of pure GTBE required, the composition of the GTBE mixture to be distilled and the vacuum distillation setup configuration and settings).

In addition, a commercially available GTBE-based blend sold as an octane booster was also tested for comparative purposes. This GTBE-based blend was tested and was found to include mono-GTBE, di-GTBE, tri-GTBE and a fair amount of trimethylpentene (TMP), with a mono-GTBE content of 4.6 wt%. The following table shows the breakdown of the composition according to GC analyses:

| Compound | GC analysis 1 (wt%) | GC analysis 2 (wt%) |
|---|---|---|
| 1-TMP | 1.1 | 1.3 |
| 2-TMP | 19.5 | 20.8 |
| 1,2,3-GTBE | 0 | 0 |
| 1,3-GTBE | 19.2 | 17.0 |
| 1,2-GTBE | 53.5 | 54.2 |
| 1-GTBE | 4.6 | 4.6 |
| 2-GTBE | 0 | 0 |

### Experimentation 9

Experiments were performed to determine the densities and viscosities of pure mono-GTBE, pure di-GTBE and pure tri-GTBE as a function of temperature. Figure 28 presents the densities of pure mono-GTBE, pure di-GTBE and pure tri-GTBE respectively as a function of temperature. For example, it was observed that the density of mono-GTBE is highest among the different GTBEs, and that the density of tri-GTBE is lowest among the different GTBEs. Figure 29 presents the dynamic viscosities of pure mono-GTBE, pure di-GTBE and pure tri-GTBE respectively as a function of temperature. For example, it was observed that the dynamic viscosity of mono-GTBE is highest among the different GTBEs, and that the dynamic viscosity of tri-GTBE is lowest among the different GTBEs.

## Claims

1. An installation for production of multiple Glycerol Tert-Butyl Ethers (GTBE) blends, comprising:
a reaction vessel comprising:
a reaction chamber for accommodating the production of GTBE;
at least one reactant inlet in fluid communication with the reaction chamber for supplying glycerol and isobutene therein;
a product outlet in fluid communication with the reaction chamber
for withdrawing the GTBE blend; and
a regulator configured for regulating conditions of the reaction vessel in alternating fashion between a first mode and a second mode, the first mode causing production of a mono-shifted GTBE blend and the second mode causing production of a di- and/or tri-shifted GTBE blend,
wherein the regulator is configured to modify a residence time of reactants in the reaction chamber, and wherein the regulator is coupled to the product outlet to regulate withdrawal of the GTBE blend.

2. The installation of claim 1, wherein the regulator is configured to modify molar ratios of the glycerol and the isobutene supplied to the reaction chamber, and wherein the regulator is coupled to the at least one reactant inlet to regulate the molar ratios.

3. The installation of claim 1 or 2, wherein the regulator is configured to modify a reaction temperature in the reaction chamber.

4. The installation of any one of claims 1 to 3, further comprising:
a first storage vessel and a second storage vessel coupled to the product outlet of the reaction vessel, and
flow direction system adapted to control flow of the GTBE blend into the first or second storage vessel respectively in the first and second modes.

## Patentansprüche

1. Anlage zur Herstellung mehrerer Glycerin-tert-butylether (GTBE)-Mischungen, umfassend:
ein Reaktionsgefäß, umfassend:
eine Reaktionskammer zum Unterbringen der Herstellung von GTBE;
wenigstens einen Reaktanteneinlass in Fluidverbindung mit der Reaktionskammer zum Zuführen von Glycerin und Isobuten darin;
einen Produktauslass in Fluidverbindung mit der Reaktionskammer
zum Entnehmen der GTBE-Mischung; und
einen Regler, konfiguriert zum Regulieren von Bedingungen des Reaktionsgefäßes abwechselnd zwischen einem ersten Modus und einem zweiten Modus, wobei der erste Modus die Herstellung einer Mono-Shift-GTBE-Mischung bewirkt und der zweite Modus die Herstellung einer Di- und/oder Tri-Shift-GTBE-Mischung bewirkt, wobei der Regler konfiguriert ist,
eine Verweilzeit von Reaktanten in der Reaktionskammer zu modifizieren, und wobei der Regler mit dem Produktauslass gekoppelt ist, um die Entnahme der GTBE-Mischung zu regulieren.

2. Anlage nach Anspruch 1, wobei der Regler konfiguriert ist, Molverhältnisse des Glycerins und des Isobutens, zugeführt der Reaktionskammer, zu modifizieren, und wobei der Regler an den wenigstens einen Reaktanteneinlass gekoppelt ist, um die Molverhältnisse zu regulieren.

3. Anlage nach Anspruch 1 oder 2, wobei der Regler konfiguriert ist, eine Reaktionstemperatur in der Reaktionskammer zu modifizieren.

4. Anlage nach einem der Ansprüche 1 bis 3, ferner umfassend: ein erstes Aufbewahrungsgefäß und ein zweites Aufbewahrungsgefäß, gekoppelt an den Produktauslass des Reaktionsgefäßes, und ein Strömungsrichtungssystem, angepasst, um die Strömung der GTBE-Mischung in das erste oder zweite Aufbewahrungsgefäß im ersten bzw. zweiten Modus zu steuern.

## Revendications

1. Une installation de production de multiples mélanges d'éthers tert-butyliques de glycérol (GTBE) comprenant :
une cuve de réaction comprenant :
une chambre de réaction pour accueillir la production de GTBE ;
au moins une entrée de réactif en communication fluidique avec la chambre de réaction pour y fournir du glycérol et de l'isobutène ;
une sortie de produit en communication fluidique avec la chambre de
réaction pour retirer le mélange GTBE ; et
un régulateur configuré pour réguler les conditions de la cuve de réaction dans un mode alterné entre un premier mode et un deuxième mode, le premier mode provoquant la production d'un mélange de mono-GTBE décalé et le deuxième mode provoquant la production d'un mélange de di-GTBE et de tri-GTBE décalé, dans lequel le régulateur est configuré pour modifier un le temps de séjour des réactifs dans la chambre de réaction, et dans lequel le régulateur est couplé à la sortie de produit pour réguler le retrait du mélange GTBE.

2. Installation selon la revendication 1, dans laquelle le régulateur est configuré pour modifier les rapports molaires du glycérol et de l'isobutène fournis à la chambre de réaction, et dans laquelle le régulateur est couplé à la au moins une entrée de réactif pour réguler les rapports molaires.

3. Installation selon la revendication 1 ou 2, dans laquelle le régulateur est configuré pour modifier une température de réaction dans la chambre de réaction.

4. Installation selon l'une quelconque des revendications 1 à 3, comprenant en outre :
un premier récipient de stockage et un deuxième récipient de stockage couplés à la sortie de produit de la cuve de réaction, et
un système de direction d'écoulement adapté pour contrôler l'écoulement du mélange de GTBE dans le premier ou le deuxième récipient de stockage respectivement dans les premier et deuxième modes.
